# EUROPEAN PATENT APPLICATION

(11) **EP 4 775 123 A1**
(43) Date of publication of application: **15.07.2026**
(21) Application number: 25151177.0
(22) Date of filing: 10.01.2025
(51) Int. Cl.: A61B 1/00, A61B 1/05, A61B 1/06

(54) **ROTATABLE ENDOSCOPE PROBE**

(71) Applicant: Shenzhen Huatuo Global Trade Co., Ltd., Shenzhen (CN)
(72) Inventor: ZHONG, Qianhua, Yichun City, Jiangxi Province (CN)
(74) Representative: Metida

(57) **Abstract**

A rotatable endoscope probe includes a camera device and a rotary control device. The rotary control device includes a fixed assembly and a rotatable assembly. The rotatable assembly is able to rotate relative to the fixed assembly. The rotatable assembly includes a connector. The rotatable assembly is connected to the camera device through the connector. The rotatable assembly drives the camera device to rotate by driving the connector. By the arrangement of the above structure, during use, the rotatable assembly in the rotary control device is connected to the camera device through the connector. The rotatable assembly drives the camera device to rotate by driving the connector, so that the field of view of observation of the camera device is increased. The blind spot of the field of view of the endoscope probe is reduced. The user experience is effectively ensured. The detection efficiency and accuracy are further improved.

## Description

### TECHNICAL FIELD

The present disclosure relates to the technical field of pipeline endoscopes, and in particular, to a rotatable endoscope probe.

### BACKGROUND OF THE INVENTION

Pipelines, as an economical, efficient, and safe material transportation product, have always been paid attention to. At present, pipeline endoscopes on the market can be used for internal inspection and observation of pipelines that are at a high temperature, toxic, and radioactive, and cannot be directly observed with human eyes, such as, to automobiles, aviation engines, pipelines, and mechanical parts. The pipeline endoscope can achieve non-destructive testing without removal or damaging the assembly and shutting down equipment, and can be used for video detection on internal welds, corrosion, blockage, differences, foreign matters, and the like in ventilation ducts, air conditioner ducts, water pipes, and industrial pipelines.

If an endoscope is used to observe and detect a situation inside a pipeline, when an endoscope probe extends into the pipeline, a blind spot of a field of view is easily caused on a side surface of the endoscope probe, which makes it impossible to effectively inspect and observe the situation of the pipeline on the side surface of the endoscope probe.

Therefore, a rotatable endoscope probe can effectively solve the above-mentioned problems.

### SUMMARY OF THE INVENTION

To overcome the shortcomings in the prior art, the present disclosure provides a rotatable endoscope probe, which has a simple structure and can further reduce a blind spot of a field of view on a side surface of the endoscope probe and effectively ensure the user experience, which further improves the detection efficiency and accuracy.

A rotatable endoscope probe, includes:
a camera device, wherein the camera device includes a first camera assembly and a second camera assembly; the first camera assembly is arranged at an axial position of the rotatable endoscope probe, and the second camera assembly is arranged at a radial position of the rotatable endoscope probe;
a rotary control device, wherein the rotary control device includes a fixed assembly and a rotatable assembly; the rotatable assembly is able to rotate relative to the fixed assembly; the rotatable assembly includes a connector; the rotatable assembly is connected to the camera device through the connector; the rotatable assembly drives the camera device to rotate by driving the connector; and
a driving device, wherein the driving device provides power to the rotatable assembly.

As an improvement of the present disclosure, the rotatable assembly includes a wire organizer; the fixed assembly includes a wire harness; the wire organizer is electrically connected to the wire harness; and the wire organizer is rotatable relative to the wire harness.

As an improvement of the present disclosure, the first camera assembly and the second camera assembly remain relatively stationary during rotation; a field of view of observation of the first camera assembly remains essentially unchanged; and a field of view of observation of the second camera assembly changes with rotation.

As an improvement of the present disclosure, the connector includes a camera connection end; the camera device includes a rotating shell; and the camera connection end is clamped with the rotating shell.

As an improvement of the present disclosure, the connector includes a rotary connection end; the wire organizer includes a power output end; and the rotary connection end and the power output end are clamped with each other.

As an improvement of the present disclosure, the driving device includes a motor; the motor includes an output shaft; the wire organizer includes a power input end; and the power input end is clamped with the output shaft.

As an improvement of the present disclosure, the wire organizer includes a plurality of conducting rings; the wire harness includes a plurality of conducting wires; and outer sides of the conducting rings resist against the conducting wires to maintain the electrical connection between the wire organizer and the wire harness.

As an improvement of the present disclosure, the rotatable endoscope probe further includes a rotatable conductor wire, inner sides of the conducting rings are electrically connected to the rotatable conductor wire; and when the rotatable assembly rotates, the rotatable conductor wire is stationary relative to the rotatable assembly.

As an improvement of the present disclosure, the rotatable endoscope probe further includes a fixed conductor wire, the conducting wires are electrically connected to the fixed conductor wire; and when the rotatable assembly rotates, the fixed conductor wire rotates relative to the rotatable assembly.

As an improvement of the present disclosure, the fixed assembly further includes a wiring harness sleeve; the wiring harness includes a first wiring harness unit and a second wiring harness unit; the conducting wires are arranged on inner sides of both the first wire harness unit and the second wire harness unit; and the wire harness sleeve is configured to restrict radial movements of the first wire harness unit and the second wire harness unit, so as to keep the outer sides of the conducting rings resisting against the conducting wires.

As an improvement of the present disclosure, the connector includes a conductor wire channel, and the rotary connection end and the camera connection end are communicated with each other through the conductor wire channel.

As an improvement of the present disclosure, the first camera assembly includes an axial camera and a front illuminator associated with the axial camera; and the second camera assembly includes a radial camera and a side illuminator associated with the radial camera.

As an improvement of the present disclosure, the front illuminator is arranged around the axial camera, and six front illuminators are included.

As an improvement of the present disclosure, the side illuminator is arranged in a manner of being adjacent to the radial camera; and one side illuminator is included.

As an improvement of the present disclosure, the rotating shell includes a connection port; the connection port includes an outer clamping portion; and the camera connection end is clamped with the outer clamping portion.

As an improvement of the present disclosure, the connection port includes an inner channel portion; the camera device includes an integrated circuit board; and the rotatable conductor wire passes through the inner channel portion and is electrically connected to the integrated circuit board to supply power to the first camera assembly and the second camera assembly.

As an improvement of the present disclosure, the camera device includes a camera shell; and the rotating shell is in threaded connection with the camera shell.

As an improvement of the present disclosure, the rotatable endoscope probe further includes a connection shell, an intermediate shell, and an end shell, the connection shell is rotatably connected to the rotating shell; the connection shell is in threaded connection with the intermediate shell; and the intermediate shell is in threaded connection with the end shell.

As an improvement of the present disclosure, the rotatable endoscope probe further includes a control circuit board, the control circuit board is configured to control rotation of the motor.

As an improvement of the present disclosure, the driving device further includes a motor sleeve; the motor sleeve is provided with a conductor wire slot; and the conductor wire slot is configured to accommodate the fixed conductor wire.

Beneficial effects: By the arrangement of the above structure, during use, the rotatable assembly in the rotary control device is connected to the camera device through the connector. The rotatable assembly drives the camera device to rotate by driving the connector, so that the field of view of observation of the camera device is increased; the blind spot of the field of view of the endoscope probe is reduced; the user experience is effectively ensured; and the detection efficiency and accuracy are further improved.

### BRIEF DESCRIPTION OF DRAWINGS

In order to explain the technical solutions of the embodiments of the present disclosure more clearly, the following will briefly introduce the accompanying drawings used in the embodiments. The drawings in the following description are only some embodiments of the present disclosure. Those of ordinary skill in the art can obtain other drawings based on these drawings without creative work. In addition, the accompanying drawings are not drawn to a scale of 1:1, and the relative dimensions of the various elements are only shown as examples in the diagrams, not necessarily drawn to a true scale.

The present disclosure is further described below in detail in combination with the accompanying drawings and embodiments.
FIG. 1 is a schematic diagram of an entire structure of a rotatable endoscope probe according to the present disclosure;
FIG. 2 is a schematic exploded diagram of a rotatable endoscope probe according to the present disclosure;
FIG. 3 is a schematic cross-sectional diagram of an entire structure of a rotatable endoscope probe according to the present disclosure;
FIG. 4 is a schematic exploded diagram of a control device 200 of a rotatable endoscope probe according to the present disclosure;
FIG. 5 is a schematic exploded diagram of a fixed assembly 210 of a rotatable endoscope probe according to the present disclosure;
FIG. 6 is a schematic exploded diagram of a rotatable assembly 220 of a rotatable endoscope probe according to the present disclosure;
FIG. 7 is a schematic diagram of an enlarged structure of a connector 221 of a rotatable endoscope probe according to the present disclosure;
FIG. 8 is a schematic diagram of an enlarged structure of a motor sleeve 320 of a rotatable endoscope probe according to the present disclosure;
FIG. 9 is a schematic diagram of an enlarged structure of a rotating shell 130 and a connection shell 600 of a rotatable endoscope probe according to the present disclosure; and
FIG. 10 is a schematic cross-sectional structural diagram of FIG. 9.

### DETAILED DESCRIPTION OF THE INVENTION

To make the aforementioned objectives, features, and advantages of the present disclosure more comprehensible, specific implementations of the present disclosure are described in detail below in conjunction with the accompanying drawings. In the following description, numerous specific details are set forth to provide a thorough understanding of the present disclosure. The present disclosure may, however, be embodied in many forms different from that described here. A person skilled in the art can make similar improvements without departing from the connotation of the present disclosure. Therefore, the present disclosure is not limited by the specific embodiments disclosed below.

In the description of the present disclosure, It is to be understood that, The terms "center", "longitudinal", "transverse", "upper", "lower", "front", "rear", "left", "right", "vertical", "horizontal", "top", "bottom", "inner", "outer", "clockwise", "counterclockwise", and the like indicate azimuth or positional relationships based on the azimuth or positional relationships shown in the drawings, For purposes of convenience only of describing the present disclosure and simplifying the description, Rather than indicating or implying that the indicated device or element must have a particular orientation, be constructed and operated in a particular orientation, therefore, not to be construed as limiting the present disclosure.

In addition, the terms "first" and "second" are used for descriptive purposes only, while not to be construed as indicating or implying relative importance or implicitly specifying the number of technical features indicated thereby, features defining "first," "second," and "second" may explicitly or implicitly include one or more of the described features. In the description of the present disclosure, "multiple" means two or more unless explicitly specified otherwise.

In addition, the terms "install", "arrange", "provide", "connect" and "couple" should be understood broadly. For example, it can be a fixed connection, a detachable connection, an integral structure, a mechanical connection, an electrical connection, a direct connection, an indirect connection through an intermediate medium, or a communication between two devices, elements or components. For ordinary technical personnel in this field, the specific meanings of the above terms in present disclosure can be understood based on specific circumstances.

In the present disclosure, unless specific regulation and limitation otherwise, the first feature "onto" or "under" the second feature may include the direct contact of the first feature and the second feature, or may include the contact of the first feature and the second feature through other features between them instead of direct contact. Moreover, the first feature "onto", "above" and "on" the second feature includes that the first feature is right above and obliquely above the second feature, or merely indicates that the horizontal height of the first feature is higher than the second feature. The first feature "under", "below" and "down" the second feature includes that the first feature is right above and obliquely above the second feature, or merely indicates that the horizontal height of the first feature is less than the second feature.

It should be noted that when an element is referred to as being "fixed to" another element, the element can be directly on another component or there can be a centered element. When an element is considered to be "connected" to another element, the element can be directly connected to another element or there may be a centered element. The terms "inner", "outer", "left", "right", and similar expressions used herein are for illustrative purposes only and do not necessarily represent the only implementation.

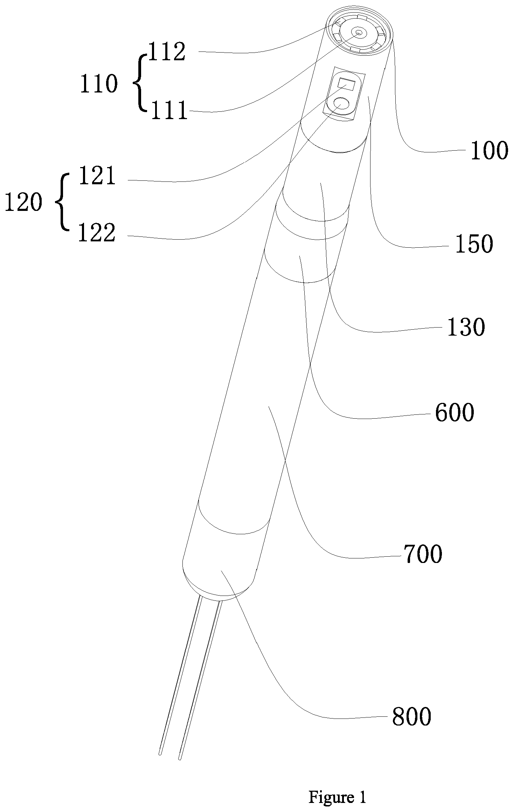

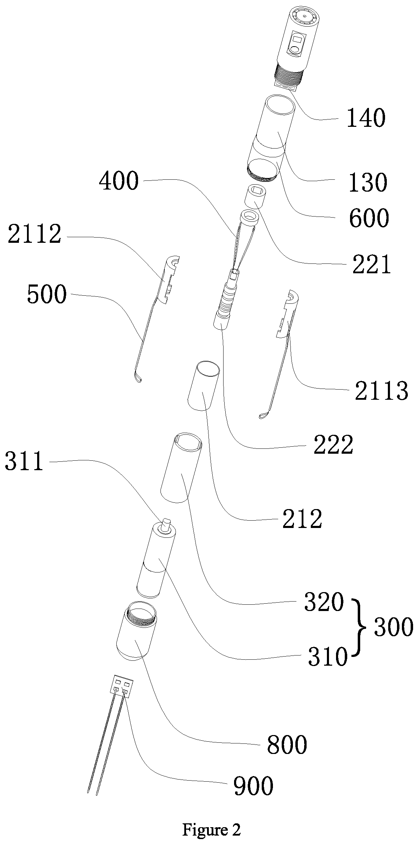

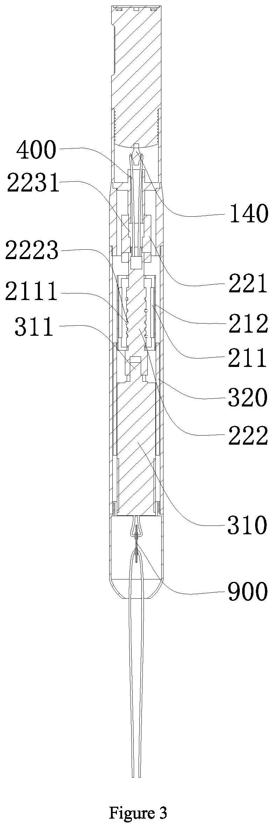

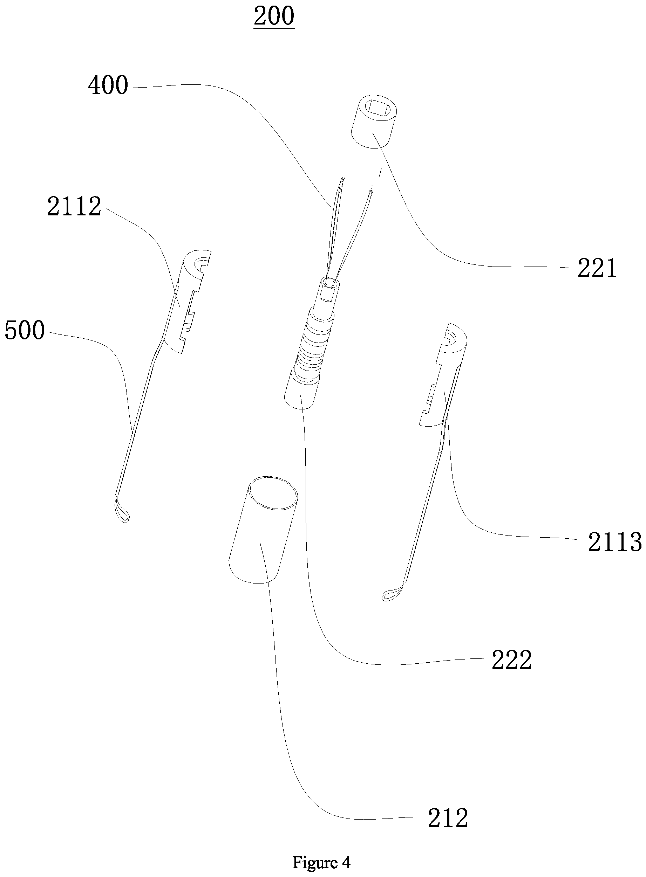

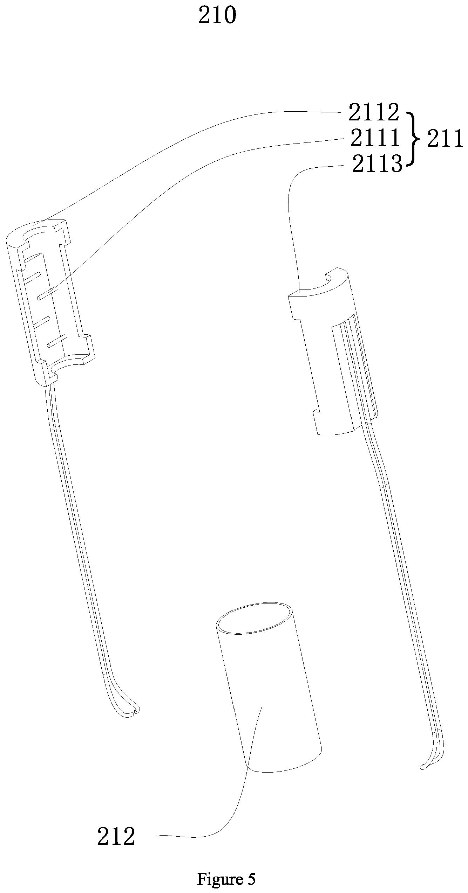

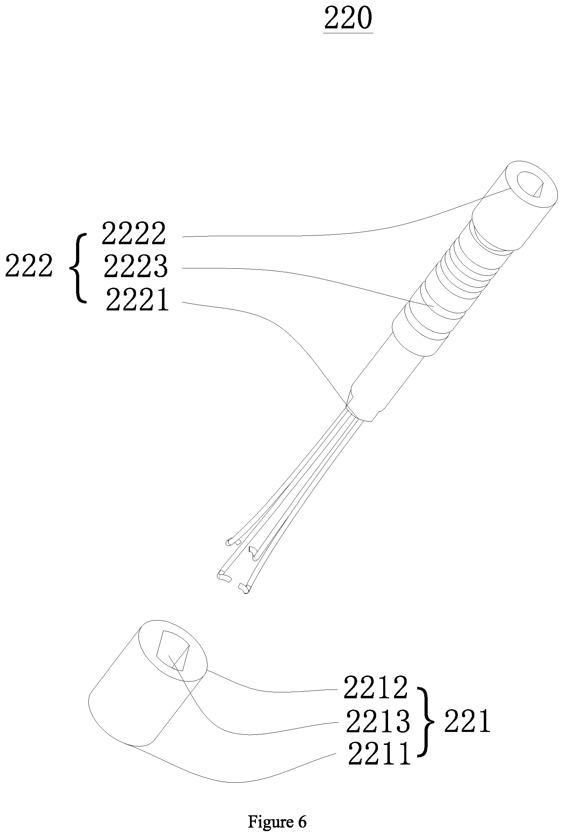

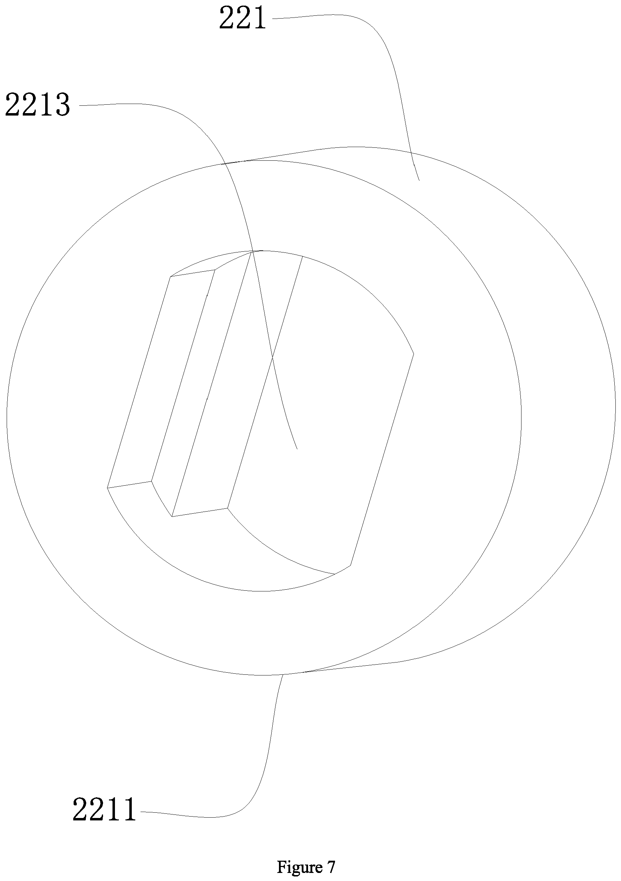

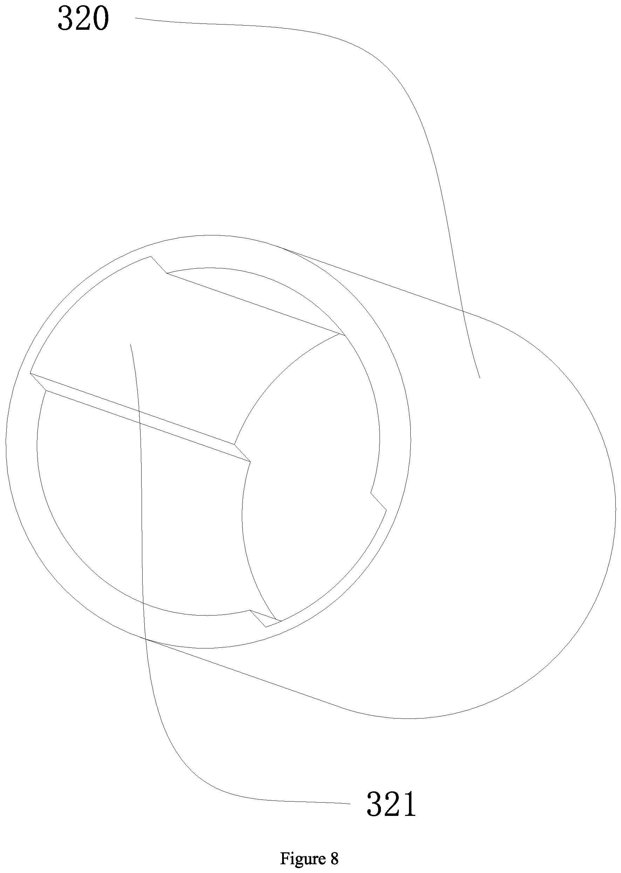

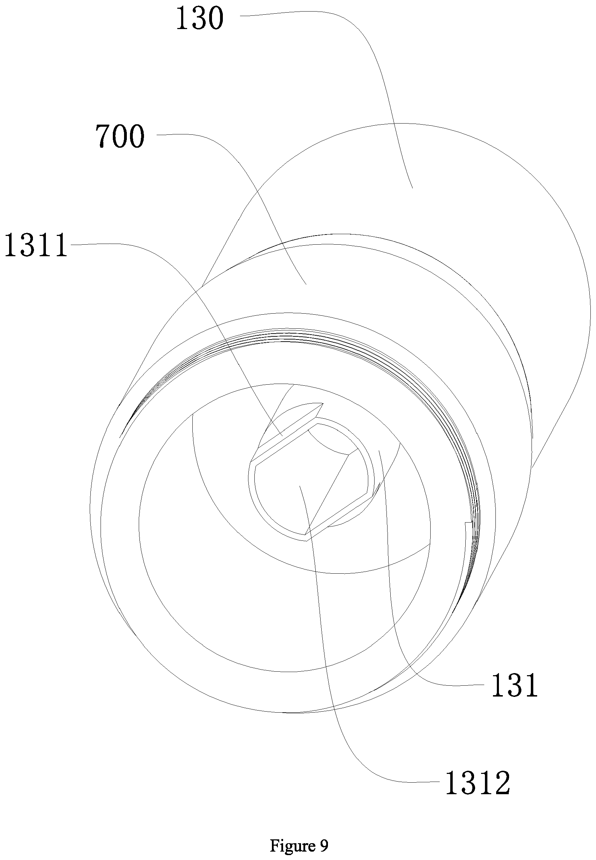

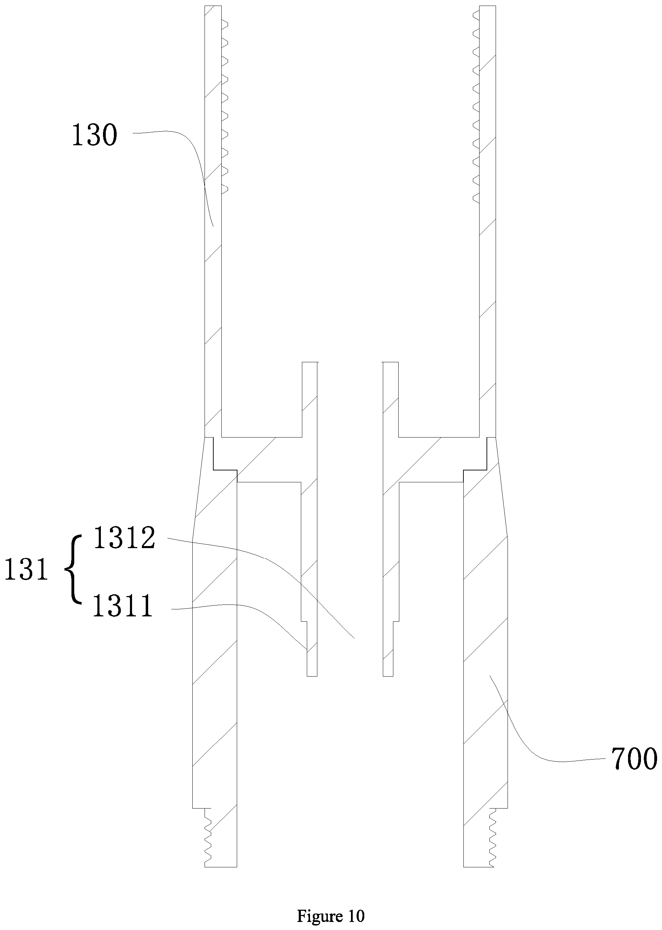

Referring to FIG. 1 to FIG. 10, a rotatable endoscope probe includes:
a camera device 100, wherein the camera device 100 includes a first camera assembly 110 and a second camera assembly 120; the first camera assembly 110 is arranged at an axial position of the rotatable endoscope probe, and the second camera assembly 120 is arranged at a radial position of the rotatable endoscope probe;
a rotary control device 200, wherein the rotary control device 200 includes a fixed assembly 210 and a rotatable assembly 220; the rotatable assembly 220 can rotate relative to the fixed assembly 210; the rotatable assembly 220 includes a connector 221; the rotatable assembly 220 is connected to the camera device 100 through the connector 221; the rotatable assembly 220 drives the camera device 100 to rotate by driving the connector 221; and
a driving device 300, wherein the driving device 300 provides power to the rotatable assembly 220.

By the arrangement of the above structure, during use, the rotatable assembly 220 in the rotary control device 200 is connected to the camera device 100 through the connector 221. The rotatable assembly 220 drives the camera device 100 to rotate by driving the connector 221, so that the field of view of observation of the camera device 100 is increased; the blind spot of the field of view of the endoscope probe is reduced; the user experience is effectively ensured; and the detection efficiency and accuracy are further improved. Since the rotatable assembly 220 is connected to the camera device 100 through the connector 221, the camera device 100 is easy to replace when it is damaged, and only the damaged camera device 100 is replaced separately. There are fields of view of observation on a front surface and side surface of the endoscope probe, and the field of view of the endoscope probe can be switched 360 degrees with rotation, so that a situation inside a pipeline can be observed and detected more comprehensively, which further reduces a proportion of the blind spot of observation, improves the monitoring efficiency, and enhances the user experience.

In this embodiment, the rotatable assembly 220 includes a wire organizer 222; the fixed assembly 210 includes a wire harness 211; the wire organizer 222 is electrically connected to the wire harness 211; and the wire organizer 222 is rotatable relative to the wire harness 211. By the arrangement of the above structure, during use, when the wire organizer 222 rotates relative to the wire harness 211, the wire organizer 222 is kept being electrically connected to the wire harness 211, thereby avoiding entanglement between a fixed conductor wire 500 electrically connected to the wire organizer 222 and a rotatable conductor wire 400 electrically connected to the wire harness 211 during rotation.

In this embodiment, the first camera assembly 110 and the second camera assembly 120 remain relatively stationary during rotation; a field of view of observation of the first camera assembly 110 remains essentially unchanged; and a field of view of observation of the second camera assembly 120 changes with rotation. By the arrangement of the above structure, there are fields of view of observation on a front surface and side surface of the endoscope probe, and the field of view of observation of the first camera assembly 110 of the endoscope probe remains basically unchanged, to ensure the stability of the field of view of observation on the front surface of the endoscope probe. The field of view of observation of the second camera assembly 120 can be switched 360 degrees with rotation, so that a situation inside a pipeline can be observed and detected more comprehensively, which further reduces a proportion of the blind spot of observation, improves the monitoring efficiency, and enhances the user experience.

In this embodiment, the connector 221 includes a camera connection end 2211; the camera device 100 includes a rotating shell 130; and the camera connection end 2211 is clamped with the rotating shell 130. By the arrangement of the above structure, during use, the camera connection end 2211 is connected to the rotating shell 130, which facilitates assembling and replacement. When the product is partially damaged, the product does not need to be replaced as a whole, only a damaged part is replaced, which prolongs the overall service life of the product and further enhances the user experience.

In this embodiment, the connector 221 includes a rotary connection end 2212; the wire organizer 222 includes a power output end 2221; and the rotary connection end 2212 and the power output end 2221 are clamped with each other. By the arrangement of the above structure, during use, the rotary connection end 2212 and the power output end 2221 are clamped with each other, so that the wire organizer 222 can drive the connector 221 to rotate and ultimately drive the camera device 100 to rotate, thus switching the field of view of observation on the side surface of the endoscope probe. This further adjusts the field of view of observation, improves the accuracy of observation, and enhances the user experience.

In this embodiment, the driving device 300 includes a motor 310; the motor 310 includes an output shaft 311; the wire organizer 222 includes a power input end 2222; and the power input end 2222 is clamped with the output shaft 311. By the arrangement of the above structure, during use, the motor 310 is clamped with the power input end 2222 through the output shaft 311, to drive the wire organizer 222 to rotate. Further, the rotary connection end 2212 and the power output end 2221 are clamped with each other, so that the wire organizer 222 can drive the connector 221 to rotate and ultimately drive the camera device 100 to rotate through the connector 221, thus switching the field of view of observation of the radial camera 121 of the endoscope probe. This adjusts the field of view of observation, improves the accuracy of observation, and further reduces the blind spot of observation to achieve a better detection effect.

In this embodiment, the wire organizer 222 includes a plurality of conducting rings 2223; the wire harness 211 includes a plurality of conducting wires 2111; and outer sides of the conducting rings 2223 resist against the conducting wires 2111 to maintain the electrical connection between the wire organizer 222 and the wire harness 211. By the arrangement of the above structure, during use, the outer sides of the conducting rings 2223 are sunken inwards. The conducting wires 2111 have elasticity. When the outer sides of the conducting rings 2223 resist against the conducting wires 2111, the outer sides of the conducting rings 2223 and the conducting wires 2111 are clamped at the inwards sunken positions on the outer sides of the conducting rings 2223 in a resisting manner. The conducting wires 2111 generate elastic deformation, so that the resisting positions of the outer sides of the conducting rings 2223 and the conducting wires 2111 have resisting force, and the outer sides of the conducting rings 2223 and the conducting wires 2111 still can keep a resisting state during relative rotation, thereby ensuring the stability of electrical connection.

In this embodiment, the rotatable endoscope probe further includes a rotatable conductor wire 400. Inner sides of the conducting rings 2223 are electrically connected to the rotatable conductor wire 400; and when the rotatable assembly 220 rotates, the rotatable conductor wire 400 is stationary relative to the rotatable assembly 220. By the arrangement of the above structure, during use, when the rotatable assembly 220 rotates, the rotatable conductor wire 400 synchronously rotates, thereby keeping the rotatable conductor wire 400 and the rotatable assembly 220 relatively stationary during the rotation, avoiding entanglement of the rotatable conductor wire 400 due to the rotation. Thus, this reduces the probability of damage to the rotatable conductor wire 400, further prolongs the service life of the product, and enhances the user experience.

In this embodiment, the rotatable endoscope probe further includes a fixed conductor wire 500; the conducting wires 2111 are electrically connected to the fixed conductor wire 500; and when the rotatable assembly 220 rotates, the fixed conductor wire 500 rotates relative to the rotatable assembly 220. By the arrangement of the above structure, during use, when the rotatable assembly 220 rotates, the fixed conductor wire 500 keeps stationary, thereby keeping the fixed conductor wire 500 and the rotatable assembly 220 rotating during the rotation. The fixed conductor wire 500 and the fixed assembly 210 are kept relatively stationary, thereby avoiding entanglement of the fixed conductor wire 500 due to the rotation. Thus, this reduces the probability of damage to the fixed conductor wire 500, further prolongs the service life of the product, and enhances the user experience.

In this embodiment, the fixed assembly 210 further includes a wiring harness sleeve 212; the wiring harness 211 includes a first wiring harness unit 2112 and a second wiring harness unit 2113; the conducting wires 2111 are arranged on inner sides of both the first wire harness unit 2112 and the second wire harness unit 2113; and the wire harness sleeve 212 is configured to restrict radial movements of the first wire harness unit 2112 and the second wire harness unit 2113, so as to keep the outer sides of the conducting rings 2223 resisting against the conducting wires 2111. By the arrangement of the above structure, during use, the first wire harness unit 2112 and the second wire harness unit 2113 are clamped with each other, and a portion of an axis section of the wire organizer 222 including the conducting wires 2111 is wrapped around the inner sides of the first wire harness unit 2112 and the second wire harness unit 2113, and the conducting wires 2111 are precisely kept resisting against the outer sides of the conducting rings 2223. When the conducting wires 2111 resist against the outer sides of the conducting rings 2223, there is elastic force between the conducting wires 2111 and the outer sides of the conducting rings 2223, which causes the first wire harness unit 2112 and the second wire harness unit 2113 which are clamped with each other to have a radial separation trend. Therefore, the wire harness sleeve 212 is provided to limit the radial movement of the first wire harness unit 2112 and the second wire harness unit 2113 and neutralize the elastic force on the first wire harness unit 2112 and the second wire harness unit 2113. The conducting wires 2111 can be stably kept resisting against the outer sides of the conducting rings 2223, thus improving the stability of the electrical connection and further enhancing the user experience.

In this embodiment, the connector 221 includes a conductor wire channel 2213, and the rotary connection end 2212 and the camera connection end 2211 are communicated with each other through the conductor wire channel 2213. By the arrangement of the above structure, during use, the rotatable conductor wire 400 can pass through the conductor wire channel 2213 to supply power to the camera device 100, thereby saving the mounting space, cleverly protecting the rotatable conductor wire 400, reducing the probability of wear of the rotatable conductor wire 400, prolonging the overall service life of the product, and enhancing the user experience.

In this embodiment, the first camera assembly 110 includes an axial camera 111 and a front illuminator 112 associated with the axial camera; and the second camera assembly 120 includes a radial camera 121 and a side illuminator 122 associated with the radial camera. By the arrangement of the above structure, during use, the front illuminator 112 can illuminate a field-of-view range observed by the axial camera 111, so that light within the field-of-view range observable by the axial camera 111 is sufficient. The side illuminator 122 can illuminate a field-of-view range observable by the radial camera 121, so that light within the field-of-view range observable by the radial camera 121 is sufficient. By the arrangement of the front illuminator 112 and the side illuminator 122, the light within the field-of-view range can be sufficient, which can better observe a situation inside a pipeline and enhances the user experience.

In this embodiment, the front illuminator 112 is arranged around the axial camera 111, and six front illuminators 112 are included. The side illuminator 122 is arranged in a manner of being adjacent to the radial camera 121; and one side illuminator 122 is included. By the arrangement of the above structure, during use, the axial camera 111 with a large field of view is provided with the plurality of front illuminators 112, which fully ensures sufficient light within the observable field of view.

In this embodiment, the rotating shell 130 includes a connection port 131; the connection port 131 includes an outer clamping portion 1311; and the camera connection end 2211 is clamped with the outer clamping portion 1311. By the arrangement of the above structure, during use, the camera connection end 2211 and the outer clamping portion 1311 are clamped with each other, so that the connection port 131 can drive the rotating shell 130 to rotate, thereby driving the radial camera 121 to rotate and adjust the field-of-view range of the radial camera 121.

In this embodiment, the connection port 131 includes an inner channel portion 1312; the camera device 100 includes an integrated circuit board 140; and the rotatable conductor wire 400 passes through the inner channel portion 1312 and is electrically connected to the integrated circuit board 140 to supply power to the first camera assembly 110 and the second camera assembly 120. By the arrangement of the above structure, during use, the rotatable conductor wire 400 can pass through the inner channel portion 1312 to supply power to the camera device 100 through the integrated circuit board 140, thereby saving the mounting space, cleverly protecting the rotatable conductor wire 400, reducing the probability of wear of the rotatable conductor wire 400, prolonging the overall service life of the product, and enhancing the user experience.

In this embodiment, the camera device 100 includes a camera shell 150; and the rotating shell 130 is in threaded connection with the camera shell 150. By the arrangement of the above structure, during use, the rotating shell 130 can drive the camera shell 150 to rotate together.

In this embodiment, the rotatable endoscope probe further includes a connection shell 600, an intermediate shell 700 and an end shell 800. The connection shell 600 is rotatably connected to the rotating shell 130; the connection shell 600 is in threaded connection with the intermediate shell 700; and the intermediate shell 700 is in threaded connection with the end shell 800. By the arrangement of the above structure, during use, the connection shell 600 is rotatably connected to the rotating shell 130. The connection shell 600 is in threaded connection with the intermediate shell 700. The intermediate shell 700 is in threaded connection with the end shell 800. Therefore, when the rotatable assembly 220 rotates, the connection shell 600, the intermediate shell 700, and the end shell 800 can remain relatively stationary, and the rotating shell 130 rotates synchronously with the rotatable assembly 220.

In this embodiment, the rotatable endoscope probe further includes a control circuit board 900. The control circuit board 900 is configured to control rotation of the motor 310. By the arrangement of the above structure, during use, a speed and rotation angle of the motor can be adjusted by the control circuit board 900 according to a detection requirement, so that the field of view of observation of the camera device 100 covers a region that needs to be observed.

In this embodiment, the driving device 300 further includes a motor sleeve 320; the motor sleeve 320 is provided with a conductor wire slot 321; and the conductor wire slot 321 is configured to accommodate the fixed conductor wire 500. By the arrangement of the above structure, during use, the fixed conductor wire 500 can be arranged in a more regular and easily distinguishable manner through the oppositely arranged conductor wire slot 321 according to different types. When a line is faulted, it is convenient for repair.

As described above, one or more embodiments are provided in conjunction with the detailed description, The specific implementation of the present disclosure is not confirmed to be limited to that the description is similar to or similar to the method, the structure and the like of the present disclosure, or a plurality of technical deductions or substitutions are made on the premise of the conception of the present disclosure to be regarded as the protection of the present disclosure.

## Claims

1. A rotatable endoscope probe, comprising:
a camera device, wherein the camera device comprises a first camera assembly and a second camera assembly; the first camera assembly is arranged at an axial position of the rotatable endoscope probe, and the second camera assembly is arranged at a radial position of the rotatable endoscope probe;
a rotary control device, wherein the rotary control device comprises a fixed assembly and a rotatable assembly; the rotatable assembly is able to rotate relative to the fixed assembly; the rotatable assembly comprises a connector; the rotatable assembly is connected to the camera device through the connector; the rotatable assembly drives the camera device to rotate by driving the connector; and
a driving device, wherein the driving device provides power to the rotatable assembly.

2. The rotatable endoscope probe according to claim 1, wherein the rotatable assembly comprises a wire organizer; the fixed assembly comprises a wire harness; the wire organizer is electrically connected to the wire harness; and the wire organizer is rotatable relative to the wire harness.

3. The rotatable endoscope probe according to claim 1, wherein the first camera assembly and the second camera assembly remain relatively stationary during rotation; a field of view of observation of the first camera assembly remains essentially unchanged; and a field of view of observation of the second camera assembly changes with rotation.

4. The rotatable endoscope probe according to claim 2, wherein the connector comprises a camera connection end; the camera device comprises a rotating shell; and the camera connection end is clamped with the rotating shell.

5. The rotatable endoscope probe according to claim 4, wherein the connector comprises a rotary connection end; the wire organizer comprises a power output end; and the rotary connection end and the power output end are clamped with each other.

6. The rotatable endoscope probe according to claim 5, wherein the driving device comprises a motor; the motor comprises an output shaft; the wire organizer comprises a power input end; and the power input end is clamped with the output shaft.

7. The rotatable endoscope probe according to claim 4, wherein the wire organizer comprises a plurality of conducting rings; the wire harness comprises a plurality of conducting wires; and outer sides of the conducting rings resist against the conducting wires to maintain the electrical connection between the wire organizer and the wire harness.

8. The rotatable endoscope probe according to claim 7, further comprising a rotatable conductor wire, wherein inner sides of the conducting rings are electrically connected to the rotatable conductor wire; and when the rotatable assembly rotates, the rotatable conductor wire is stationary relative to the rotatable assembly.

9. The rotatable endoscope probe according to claim 7, further comprising a fixed conductor wire, wherein the conducting wires are electrically connected to the fixed conductor wire; and when the rotatable assembly rotates, the fixed conductor wire rotates relative to the rotatable assembly.

10. The rotatable endoscope probe according to claim 7, wherein the fixed assembly further comprises a wiring harness sleeve; the wiring harness comprises a first wiring harness unit and a second wiring harness unit; the conducting wires are arranged on inner sides of both the first wire harness unit and the second wire harness unit; and the wire harness sleeve is configured to restrict radial movements of the first wire harness unit and the second wire harness unit, so as to keep the outer sides of the conducting rings resisting against the conducting wires.

11. The rotatable endoscope probe according to claim 5, wherein the connector comprises a conductor wire channel, and the rotary connection end and the camera connection end are communicated with each other through the conductor wire channel.

12. The rotatable endoscope probe according to claim 3, wherein the first camera assembly comprises an axial camera and a front illuminator associated with the axial camera; and the second camera assembly comprises a radial camera and a side illuminator associated with the radial camera.

13. The rotatable endoscope probe according to claim 12, wherein the front illuminator is arranged around the axial camera, and six front illuminators are comprised.

14. The rotatable endoscope probe according to claim 12, wherein the side illuminator is arranged in a manner of being adjacent to the radial camera; and one side illuminator is comprised.

15. The rotatable endoscope probe according to claim 8, wherein the rotating shell comprises a connection port; the connection port comprises an outer clamping portion; and the camera connection end is clamped with the outer clamping portion.

## Amended claims

### Amended claims in accordance with Rule 137(2) EPC.

1. A rotatable endoscope probe for pipeline, comprising:
a camera device (100), wherein the camera device (100) comprises a first camera assembly (110) and a second camera assembly (120); the first camera assembly (110) is arranged at an axial position of the rotatable endoscope probe, and the second camera assembly (120) is arranged at a radial position of the rotatable endoscope probe;
a rotary control device (200), wherein the rotary control device (200) comprises a fixed assembly (210) and a rotatable assembly (220); the rotatable assembly (220) is able to rotate relative to the fixed assembly (210); the rotatable assembly (220) comprises a connector (221);
the rotatable assembly (220) is connected to the camera device (100) through the connector (221); the rotatable assembly (220) drives the camera device (100) as a whole to rotate by driving the connector (221); and
a driving device (300), wherein the driving device (300) provides power to the rotatable assembly (220);
wherein the driving device (300) comprises a motor (310); the motor (310) comprises an output shaft (311), the camera device (100) comprises a camera shell (150) accommodating the first camera assembly (110) and the second camera assembly (120) therein, the camera shell (150) and the rotary control device (200) are coaxial with the output shaft (311); and
wherein the first camera assembly (110) comprises an axial camera (111) facing forward and being coaxial with the camera shell (150), and the second camera assembly (120) comprises a radial camera (121) exposed at the radial position of the camera shell (150), during rotation, a field of view of observation of the first camera assembly (110) remains essentially unchanged; and a field of view of observation of the second camera assembly (120) changes with rotation.

2. The rotatable endoscope probe according to claim 1, wherein the rotatable assembly (220) comprises a wire organizer (222); the fixed assembly (210) comprises a wire harness (211); the wire organizer (222) is electrically connected to the wire harness (211); and the wire organizer(222) is rotatable relative to the wire harness (211).

3. The rotatable endoscope probe according to claim 2, wherein the connector (221) comprises a camera connection end (2211); the camera device (100) comprises a rotating shell (130); and the camera connection end (2211) is clamped with the rotating shell (130).

4. The rotatable endoscope probe according to claim 3, wherein the connector (221) comprises a rotary connection end (2212); the wire organizer (222) comprises a power output end (2221); and the rotary connection end (2212) and the power output end (2221) are clamped with each other.

5. The rotatable endoscope probe according to claim 4, wherein the wire organizer (222) comprises a power input end (2222); and the power input end (2222) is clamped with the output shaft (311).

6. The rotatable endoscope probe according to claim 3, wherein the wire organizer (222) comprises a plurality of conducting rings (2223); the wire harness (211) comprises a plurality of conducting wires (2111); and outer sides of the conducting rings (2223) resist against the conducting wires (2111) to maintain the electrical connection between the wire organizer (222) and the wire harness (211).

7. The rotatable endoscope probe according to claim 6, further comprising a rotatable conductor wire (400), wherein inner sides of the conducting rings (2223) are electrically connected to the rotatable conductor wire (400); and when the rotatable assembly (220) rotates, the rotatable conductor wire (400) is stationary relative to the rotatable assembly (220).

8. The rotatable endoscope probe according to claim 6, further comprising a fixed conductor wire (500), wherein the conducting wires (2111) are electrically connected to the fixed conductor wire (500); and when the rotatable assembly (220) rotates, the fixed conductor wire (500) rotates relative to the rotatable assembly (220).

9. The rotatable endoscope probe according to claim 6, wherein the fixed assembly (210) further comprises a wiring harness sleeve (212); the wiring harness (211) comprises a first wiring harness unit (2112) and a second wiring harness unit (2113); the conducting wires (2111) are arranged on inner sides of both the first wire harness unit (2112) and the second wire harness unit (2113); and the wire harness sleeve (212) is configured to restrict radial movements of the first wire harness unit (2112) and the second wire harness unit (2113), so as to keep the outer sides of the conducting rings (2223) resisting against the conducting wires (2111).

10. The rotatable endoscope probe according to claim 4, wherein the connector (221) comprises a conductor wire channel (2213), and the rotary connection end (2212) and the camera connection end (2211) are communicated with each other through the conductor wire channel (2213).

11. The rotatable endoscope probe according to claim 1, wherein the first camera assembly (110) further comprises a front illuminator (112) associated with the axial camera (111); and the second camera assembly (120) further comprises a side illuminator (122) associated with the radial camera (121).

12. The rotatable endoscope probe according to claim 11, wherein the front illuminator (112) is arranged around the axial camera (111), and six front illuminators (112) are comprised.

13. The rotatable endoscope probe according to claim 11, wherein the side illuminator (122) is arranged in a manner of being adjacent to the radial camera (121); and one side illuminator (122) is comprised.

14. The rotatable endoscope probe according to claim 7, wherein the rotating shell (130) comprises a connection port (131); the connection port (131) comprises an outer clamping portion (1311); and the camera connection end (2211) is clamped with the outer clamping portion (1311).
